(19) 

Europäisches Patentamt
European Patent Office
Office européen des brevets

(11) **EP 1 958 954 A1**

(12) **EUROPEAN PATENT APPLICATION**
published in accordance with Art. 158(3) EPC

(43) Date of publication:
**20.08.2008 Bulletin 2008/34**

(21) Application number: **06833736.9**

(22) Date of filing: **30.11.2006**

(51) Int Cl.:
*C07H 13/06* [(2006.01)]  *C07H 5/02* [(2006.01)]
*A61K 51/00* [(2006.01)]  *C07B 61/00* [(2006.01)]

(86) International application number:
**PCT/JP2006/323933**

(87) International publication number:
**WO 2007/066567 (14.06.2007 Gazette 2007/24)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.12.2005 JP 2005352464**

(71) Applicant: **NIHON MEDI-PHYSICS CO., LTD.**
**Tokyo 136-0075 (JP)**

(72) Inventors:
• **HIRANO, Keiichi**
  **Sodegaura-shi Chiba 299-0266 (JP)**
• **NAKAMURA, Daisaku**
  **Sodegaura-shi Chiba 299-0266 (JP)**

(74) Representative: **Wilhelms · Kilian & Partner**
**Patentanwälte**
**Eduard-Schmid-Strasse 2**
**81541 München (DE)**

(54) **PROCESS FOR PRODUCTION OF COMPOUND LABELED WITH RADIOACTIVE FLUORINE**

(57) A process for production of [18F]-TAFDG is provided, which can produce [18F]-TAFDG in a high radiofluorination yield. A process for production of an organic compound labeled with radioactive fluorine comprises the steps of: preparing a reaction solution containing [18F] fluoride ions, a phase transfer catalyst, potassium ions, 1,3,4,6-tetra-O-acetyl-2-O-trifluoromethanesulfonyl-β-D-mannopyranose as a labeling precursor compound, and a solvent; and giving a reaction condition to said reaction solution to obtain 1,3,4,6-tetra-O-acetyl-2-[18F] fluoro-2-deoxyglucose, in which said solvent contains water.

**Description**

TECHNICAL FIELD

The present invention relates a process for production of organic compounds labeled with radioactive fluorine, specifically 1,3,4,6-tetra-O-acetyl-2-[18F] fluoro-2-deoxyglucose and its deprotected product, 2-[18F] fluoro-2-deoxy-D-glucose.

BACKGROUND ART

Nuclear medicine examination represented by positron emission tomography (hereinafter referred to as PET) and single photon emission computed tomography (hereinafter referred to as SPECT) is effective in diagnosing a variety of diseases including cancers. These examination techniques involve administrating an agent labeled with a specific radioisotope (hereinafter referred to as radiopharmaceutical) to a patient followed by detecting γ-ray emitted directly or indirectly from the agent. Nuclear medicine examination is characteristic in that it has not only high specificity and sensitivity to diseases, but also an advantage of providing information on the function of lesions, compared to other examination techniques.
For example, 2-[18F]fluoro-2-deoxy-D-glucose (hereinafter referred to as 18F-FDG), one of radiopharmaceuticals used for PET examination, tends to be concentrated in areas where glucose metabolism is enhanced, thereby making it possible to specifically detect tumors in which glucose metabolism is enhanced.

Among the above-mentioned nuclear medicine examinations, PET provides images of higher quality and thus can provide images higher in diagnosis performance, compared to SPECT that has been widely used conventionally in clinical use. PET examination is, therefore, expected as a new diagnostic modality succeeding SPECT examination, and development of radiopharmaceuticals for PET examination (hereinafter referred to as PET diagnostic agent) is now carried out in many research facilities and the like. For example, various receptor mapping agents and blood flow diagnostic agents have been synthesized and are under investigation for clinical application.

The PET diagnostic agent is an agent that contains, as an effective component, a compound labeled with a positron emitting nuclide such as 11C, 15O and 18F. The most widely used compound among them is an organic compound labeled with 18F represented by 18F-FDG. There have been reported various methods for production of the representative compound, 18F-FDG, and most of them are generally classified into a method proposed by Hamacher (hereinafter referred to as Hamacher method) and an on-column method.

The Hamacher method is a method wherein [18F] are activated by evaporating a solution containing [18F], potassium carbonate and a phase transfer catalyst to dryness, then a solution in acetonirile of 1,3,4,6-tetra-O-acetyl-2-O-trifluoromethanesulfonyl-β-D-mannopyranose (hereinafter referred to as TATM) that is a labeling precursor is added and heated to give an intermediate, 1,3,4,6-tetra-O-acetyl-2-fluoro-2-deoxyglucose (hereinafter referred to as 18F-TAFDG), followed by subjecting the intermediate to a deprotection process and a purification process to give targeted 18F-FDG. On the other hand, the on-column method is a method wherein 18F-FDG is obtained by performing activation of [18F] and 18F labeling reaction in a column followed by deprotection and purification (see Patent Document 1, Non-Patent Document 1 and Non-Patent Document 2).

It is disclosed that when 18F-FDG is synthesized by the above mentioned method, complete dehydration is required in the step in which the solution containing 18F, potassium carbonate and a phase transfer catalyst is evaporated to dryness to activate 18F (see Non-Patent Document 1 and Patent Document 2).
In addition, it is disclosed that if dehyderation is insufficient in the above mentioned step of activating 18 F in the synthesis of an organic compound labeled with 18F, 18F may be hydrated, thereby reducing nucleophilicity of 18F and causing decrease of yield (see Patent Document 3).

Patent Document 1: Japanese Patent Laid-Open (Kokai) No. 6-157572.
Patent Document 2: Japanese Patent Laid-Open (Kohyo) No. 11-508923.
Patent Document 3: Japanese Patent Laid-Open (Kokai) No. 5-345731.
Non-Patent Document 1: Hamacher K., Coenen H.H., Stocklin G., "Efficient Stereospecific Synthesis of No-carrier-added-2-[18F]fluoro-2-deoxy-D-glucose Using Aminopolyether Supported Nucleophilic Substitution, "J. Nucl. Med., 1986, 27, 2, p.235-238.
Non-Patent Document 2: K. Hamacher et al., "Computer-aided Synthesis (CAS) of No-carrier-added-2-[18F]Fluoro-2-deoxy-D-glucose: an Efficient Automated System for the Aminopolyether-supported Nucleophilic Fluorination" Applied Radiation and Isotopes, (Great Britain), Pergamon Press, 1990, 41, 1, p.49-55.

DISCLOSURE OF THE INVENTION

PROBLEM TO BE SOLVED BY THE INVENTION

Among the representative methods for producing $^{18}F$-FDG, the Hamacher method has a feature such that it can achieve a relatively high yield, but nevertheless has a problem such that production yield may greatly vary in some cases. The primary cause of this variation is the variation of yield of $^{18}F$ labeling reaction, that is, the variation of production yield of $^{18}F$-TAFDG (hereinafter referred to as radiofluorination yield). Therefore, in order to stably supply $^{18}F$-FDG commercially, it is necessary to adopt a method or condition that enables stable production in high yield, and it is necessary for that purpose to establish a condition in which $^{18}F$-TAFDG can be stably produced in high yield.

The present invention has been made in light of the above situation, and has aimed at providing a process for production of $^{18}F$-TAFDG, which can achieve a high radiofluorination yield.

MEANS FOR SOLVING THE PROBLEM

As a result of diligent studies, the inventors have completed the invention by finding that the above mentioned problem can be solved and a high radiofluorination yield can be achieved by allowing a reaction solution for fluorination reaction to contain a certain amount of water.

According to the conventional knowledge, the presence of water in the reaction solution acts as a factor that inhibits $^{18}F$ fluorination reaction, and therefore it has been suggested that complete dehydration is required in the $^{18}F$ activation step (that is, a step of obtaining a mixture containing [$^{18}F$]fluoride ions, a phase transfer catalyst and potassium ions) which is performed prior to $^{18}F$ fluorination reaction in the synthesis of $^{18}F$-FDG (see Japanese Patent Laid-Open (Kohyo) No. 11-508923 and Japanese Patent Laid-Open (Kokai) No. 5-345731). On the contrary to such a conventional knowledge, we have found that the yield in the $^{18}F$ fluorination reaction can be improved and stabilized by allowing a certain amount of water to coexist in the reaction solution and thus completed the present invention.

A process for production of an organic compound labeled with radioactive fluorine according to the present invention is a process comprising the steps of:

preparing a reaction solution containing [$^{18}F$]fluoride ions, a phase transfer catalyst, potassium ions, TATM as a labeling precursor compound, and a solvent; and giving a reaction condition to the above-mentioned reaction solution to obtain $^{18}F$-TAFDG, characterized in that the above-mentioned solvent contains water.

As the above-mentioned solvent of the reaction solution, an amphipathic organic solvent can be used which is capable of dissolving TATM and contains a certain amount of water; typically a liquid mixture of acetonitrile and water can be used.

Amount of water to be contained in the solvent is preferably 500 $\mu g/g$ to 10000 $\mu g/g$, more preferably 750 $\mu g/g$ to 9500 $\mu g/g$, still more preferably 1000 $\mu g/g$ to 9000 $\mu g/g$, particularly preferably 1000 $\mu g/g$ to 3000 $\mu g/g$. The case where the amount of water contained in the solvent is too low is not preferable, because fluorination reaction does not proceed sufficiently. The case where the amount is too much is not preferable either, because decrease in fluorination reaction yield and increase in impurity are brought about.

In the production process according to the present invention, the step of preparing a reaction solution is performed typically by preparing a mixture containing [$^{18}F$]fluoride ions, a phase transfer catalyst and potassium ions beforehand, and adding TATM and a solvent to the mixture respectively. It is preferable that TATM is previously dissolved in a solvent, and then added. The step of preparing a reaction solution is not, however, limited to these methods. Water to be contained in the solvent is preferably added simultaneously with or prior to the addition of TATM. Specifically, the step of preparing a reaction solution can be performed by adding TATM as a labeling precursor compound to a system in which water or a water-containing solvent is added to the mixture of [$^{18}F$]fluoride ions, a phase transfer catalyst and potassium ions. The introduction of water to the above described system before the addition of TATM to the system enables a more stable and higher $^{18}F$ fluorination yield.

EFFECT OF THE INVENTION

It has become possible to obtain $^{18}F$-TAFDG in a high radiofluorination yield by use of the process for production of an organic compound labeled with radioactive fluorine according to the present invention.

BEST MODE FOR CARRYING OUT THE INVENTION

Hereinafter, the most preferable embodiment will be described about the process for production of an organic compound labeled with radioactive fluorine according to the present invention.

In the process for production of an organic compound labeled with radioactive fluorine according to the present invention, a mixture containing a phase transfer catalyst, [$^{18}$F]fluoride ions and potassium ions is obtained in the first place. [$^{18}$F]fluoride ions can be obtained by a known method, for example, a method in which $H_2{}^{18}O$ enriched water is used as a target and exposed to a proton bombardment. In this instance, [$^{18}$F]fluoride ions exist in the $H_2{}^{18}O$ enriched water used as a target. This $H_2{}^{18}O$ enriched water containing [$^{18}$F] fluoride ions is passed through an anion-exchange column to collect radioactive fluorine by adsorption to the column, and thus separated from the $H_2{}^{18}O$ enriched water. Then, a potassium carbonate solution is run through the column to elute the [$^{18}$F]fluoride ions, and supplemented with a phase transfer catalyst and dried to solid to obtain a mixture containing a phase transfer catalyst, [$^{18}$F] fluoride ions and potassium ions.

Amount of potassium carbonate to be used here may be about 0.3 or more in terms of molar ratio relative to the labeling precursor TATM, but should not be excessive because it causes decrease of yield unfavorably. In the most preferable aspect, concentration and amount of the potassium carbonate solution are adjusted so that the molar ratio of potassium ion to TATM is 0.3 to 4.

Various compounds having a property to form a clathrate with $^{18}$F ion may be used as a phase transfer catalyst. Specifically, various compounds used for production of organic compounds labeled with radioactive fluorine may be used; 18-crown-6-ether and other various aminopolyethers may be used. In the most preferable aspect, CRYPTOFIX 222 (trade name, manufactured by Merck Ltd.) may be used.

After a mixture containing a phase transfer catalyst, [$^{18}$F] fluoride ions and potassium ions is obtained, a reaction solution containing the mixture and the labeling precursor compound TATM, is prepared. The process according to the present invention is characterized in that the solvent in the reaction solution prepared in this step comprises water. In the most preferable aspect, this step is performed by adding water-containing acetonitrile to the above-described mixture followed by addition of TATM.

Amount of water to be contained in acetonitrile is adjusted so that concentration of water is 500 to 10000 $\mu$g/g in the prepared reaction solution. Amount of the solvent to be added is not specifically restricted as long as it is sufficient to dissolve the above-mentioned mixture, however, it must be noted that if the amount is too much, too much processing time is required for the heat and evaporation step for preparing $^{18}$F-FDG from $^{18}$F-TAFDG.

The method for adding TATM is not specially limited, but a method in which TATM is first dissolved in acetonitrile and then added is preferable from the viewpoint of operability. In this case, it is preferable that acetonitrile which is used to dissolve TATM contains water in an amount as small as possible, from the viewpoint of inhibiting decomposition of TATM.

After the completion of preparation of the reaction solution, labeling reaction is performed by giving a reaction condition to the reaction solution. The reaction condition may be a condition which is sufficient for progressing $^{18}$F fluorination reaction of TATM by means of heating or the like. In the production of $^{18}$F-TAFDG, labeling with $^{18}$F can be usually achieved by heating a vessel containing a reaction solution at 85°C for about 5 minutes in a hermetic state. $^{18}$F-TAFDG can be synthesized by this procedure.

When $^{18}$F-FDG is prepared by deprotection of $^{18}$F-TAFDG, a step may be performed in which the reaction vessel is left open after the completion of labeling reaction, and the reaction solution is heated to evaporate the solvent followed by addition of an acid and heating. In addition, the obtained $^{18}$F-FDG may be subjected to a purification process if required.

EXAMPLES

Hereinafter, the present invention will be described in further detail with reference to Examples; however, it should be understood that the details of the Examples are not intended to limit the present invention.

Examples 1-11

A target water enriched with $^{18}$O was subjected to proton bombardment to obtain [$^{18}$F]fluoride ions in a form of a target water containing [$^{18}$F]fluoride ions. This target water containing [$^{18}$F]fluoride ions was measured for the amount of radioactivity using CRC-15R (trade name, manufactured by CAPINTEC, Inc.) (denoted as the amount of radioactivity A). Then, after this target water was passed through an anion-exchange column to collect [$^{18}$F]fluoride ions by adsorption, a potassium carbonate solution was passed through the column to elute [$^{18}$F]fluoride ions. This eluate containing [$^{18}$F] fluoride ions was supplemented with a solution of CRYPTOFIX 222 (trade name, manufactured by Merck Ltd.) in acetonitrile, heated and evaporated to dryness, to obtain a mixture containing [$^{18}$F]fluoride ions, potassium ions and a phase transfer catalyst (CRYPTOFIX 222, trade name, manufactured by Merck Ltd.).

By adding 0.5 mL of acetonitrile mixed with water to the mixture, the mixture was dissolved therein so that the water content in the reaction solution was that shown in Table 1. The resultant solution was supplemented with 0.5 mL of a solution of TATM in acetonitrile (concentration: 40 mg/mL) to prepare the reaction solution. Then, the vessel containing the reaction solution was sealed and heated at 85°C for 5 minutes using a heating block to perform the labeling reaction.

After the completion of the reaction, the vessel was opened and heated further at 85°C for 5 minutes to evaporate the solvent, and measurement of radioactivity was performed using CRC-15R (trade name, manufactured by CAPINTEC, Inc.) (referred to as the amount of radioactivity B).

The obtained reaction product was dissolved in acetonitrile with a water content of 1 % (w/w) and analyzed by TLC under the following condition. The area percentage of 18F-TAFDG was considered as the radiochemical purity.

TLC analysis condition:

TLC plate: Silica Gel 60 F$_{254}$ (trade name, manufactured by Merck Ltd.)

Mobile phase: chloroform/ethyl acetate = 80/20

Detector: Rita Star (trade name, manufactured by raytest Co. Ltd.)

Using the obtained radiochemical purity and the amounts of radioactivity A and radioactivity B, the [18F]fluorination yield was determined according to the following equation (1). Here, the amounts of radioactivity A and radioactivity B corrected for decay in consideration of measurement time were used in the calculation.

$$[^{18}F]\text{fluorination yield (\%)} = \frac{B}{A} \times \left[\text{radiochemical purity of }^{18}F\text{-TAFDG}\right] \quad (1)$$

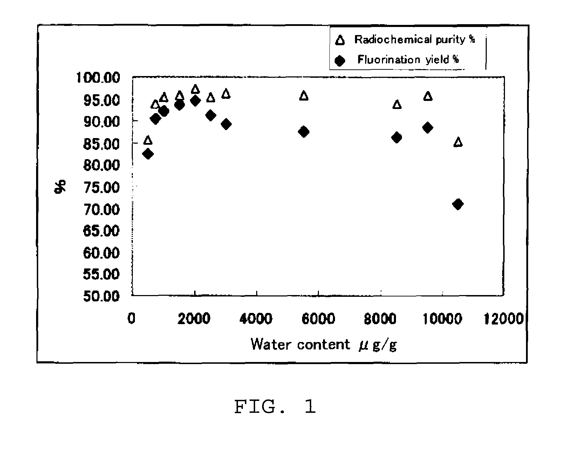

The results are shown in Table 1 and Figure 1. As shown in the table and the figure, the value of fluorination yield of 18F-TAFDG was as good as not less than 70 % when the water content in the reaction solution was between 500 to 10000 μg/g. On the other hand, it was shown that when the water content in the reaction solution is less than 500 μg/g and when more than 9500 μg/g, the fluorination yield and the 18F-TAFDG value tended to decline.

From the results described above, it was shown that 18F-TAFDG can be produced in a good fluorination yield when the water content in a reaction solution is from 500 to 10000 μg/g. Especially, the fluorination yield of not less than 85 % and the radiochemical purity of not less than 90 % were achieved when the water content was between 750 and 9500 μg/g.

Table 1

|  | Water content in the reaction solution μg/g | Radiochemical purity of 18F-TAFDG % | Fluorination yield of 18F-TAFDG % |
|---|---|---|---|
| Example 1 | 500 | 85.69 | 82.64 |
| Example 2 | 750 | 94.01 | 90.46 |
| Example 3 | 1000 | 95.33 | 92.23 |
| Example 4 | 1500 | 95.80 | 93.67 |
| Example 5 | 2000 | 97.29 | 94.56 |
| Example 6 | 2500 | 95.30 | 91.18 |
| Example 7 | 3000 | 96.27 | 89.24 |
| Example 8 | 5500 | 95.80 | 87.69 |
| Example 9 | 8500 | 93.91 | 86.41 |
| Example 10 | 9500 | 94.95 | 88.62 |
| Example 11 | 10000 | 85.50 | 71.18 |

INDUSTRIAL APPLICABILITY

The process for production of an organic compound labeled with radioactive fluorine according to the present invention can be employed in producing a radiopharmaceutical 18F-FDG, and is useful in the field of nuclear medicine examination.

BRIEF DESCRIPTION OF THE DRAWING

Figure 1 shows the relationship of the water content in the reaction solution with the radiochemical purity of 18F-

TAFDG and the fluorination yield.

**Claims**

1. A process for production of an organic compound labeled with radioactive fluorine, which comprises the steps of:

   preparing a reaction solution containing [18F] fluoride ions, a phase transfer catalyst, potassium ions, 1,3,4,6-tetra-O-acetyl-2-O-trifluoromethanesulfonyl-β-D-mannopyranose as a labeling precursor compound, and a solvent; and
   giving a reaction condition to said reaction solution to obtain 1,3,4,6-tetra-O-acetyl-2-[18F]fluoro-2-deoxyglucose; in which said solvent contains water.

2. The process for production of an organic compound labeled with radioactive fluorine, according to Claim 1, in which said solvent is a liquid mixture of water and acetonitrile.

3. The process for production of an organic compound labeled with radioactive fluorine, according to Claim 1 or 2, in which the water content in said reaction solution is from 500 to 10000 μg/g.

4. The process for production of an organic compound labeled with radioactive fluorine, according to any one of Claims 1 to 3, in which said step of preparing a reaction solution comprises adding 1,3,4,6-tetra-O-acetyl-2-O-trifluoromethanesulfonyl-β-D-mannopyranose to a system in which water or a water-containing solvent is added to a mixture of [18F]fluoride ions, a phase transfer catalyst and potassium ions.

FIG. 1

<table>
<tr><td colspan="2" align="center">**INTERNATIONAL SEARCH REPORT**</td><td>International application No.<br>PCT/JP2006/323933</td></tr>
</table>

| A. CLASSIFICATION OF SUBJECT MATTER |
|---|
| *C07H13/06*(2006.01)i, *C07H5/02*(2006.01)n, *A61K51/00*(2006.01)n, *C07B61/00* (2006.01)n |

According to International Patent Classification (IPC) or to both national classification and IPC

| B. FIELDS SEARCHED |
|---|

Minimum documentation searched (classification system followed by classification symbols)
C07H13/06, C07H5/02, A61K51/00, C07B61/00

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)
CAplus(STN), CASREACT(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 6-157572 A   (General Electric Co.),<br>03 June, 1994 (03.06.94),<br>& US 5264570 A           & CA 2101623 A1<br>& EP 588480 A1 | 1-4 |
| A | JP 5-345731 A   (NKK Corp.),<br>27 December, 1993 (27.12.93),<br>(Family: none) | 1-4 |
| A | DIKSIC, Mirko et al., Synthesis of 2-deoxy-2-fluorohexoses byfluorination of glycals in aqueous media, Carbohydrate Research, 1986, Vol.153, No.1, p.17-24 | 1-4 |

☐ Further documents are listed in the continuation of Box C.          ☐ See patent family annex.

| | |
|---|---|
| * Special categories of cited documents:<br>"A" document defining the general state of the art which is not considered   to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>05 February, 2007 (05.02.07) | Date of mailing of the international search report<br>13 February, 2007 (13.02.07) |
| Name and mailing address of the ISA/<br>Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6157572 A
- JP 11508923 A
- JP 5345731 A

**Non-patent literature cited in the description**

- **HAMACHER K. ; COENEN H.H. ; STOCKLIN G.** Efficient Stereospecific Synthesis of No-carrier-added-2-[18F]fluoro-2-deoxy-D-glucose Using Aminopolyether Supported Nucleophilic Substitution. *J. Nucl. Med.,* 1986, vol. 27 (2), 235-238
- Computer-aided Synthesis (CAS) of No-carrier-added-2-[18F]Fluoro-2-deoxy-D-glucose: an Efficient Automated System for the Aminopolyether-supported Nucleophilic Fluorination. **K. HAMACHER et al.** Applied Radiation and Isotopes, (Great Britain. Pergamon Press, 1990, vol. 41, 49-55